# EUROPÄISCHE PATENTANMELDUNG

(11) **EP 4 410 232 A1**
(43) Veröffentlichungstag der Anmeldung: **07.08.2024**
(21) Anmeldenummer: 23154817.3
(22) Anmeldetag: 03.02.2023
(51) Int. Cl.: A61B 90/70

(54) **CHIRURGISCHE REINIGUNGSVORRICHTUNG MIT ADAPTER**

(71) Anmelder: Heraeus Medical GmbH, 61273 Wehrheim (DE)
(72) Erfinder: DR. VOGT, Sebastian, 61273 Wehrheim (DE); DR. KLUGE, Thomas, 61273 Wehrheim (DE)
(74) Vertreter: Heraeus IP

(57) **Zusammenfassung**

Die Erfindung betrifft eine Vorrichtung zur chirurgischen Reinigung infizierter Implantate, welche einen Bürstenkopf, welcher einen Borstenbereich mit einer Vielzahl von Borsten aufweist; ein Zuleitungselement, das zum Transport einer Reinigungsflüssigkeit zum Bürstenkopf eingerichtet ist; ein Ableitungselement, das zur Entfernung von Flüssigkeit vom Bürstenkopf eingerichtet ist; und ein Verbindungselement, welches zur lösbaren Verbindung des Zuleitungselements und des Ableitungselements mit einer externen Lavagevorrichtung eingerichtet ist, aufweist.

## Beschreibung

### GEBIET DER ERFINDUNG

Die vorliegende Erfindung betrifft das Gebiet der Medizintechnik, insbesondere Vorrichtungen und Verfahren zur Anwendung in der chirurgischen Reinigung infizierten Gewebes. Gegenstand der Erfindung ist insbesondere eine chirurgische Reinigungsvorrichtung, die als einmalig zu verwendende Vorrichtung zur mechanischen Reinigung und Spülung von infizierten Gelenkendoprothesen im Rahmen von DAIR-Prozeduren verwendbar sein kann. Die Reinigungsvorrichtung ist zum manuellen Bürsten von Gelenkendoprothesenoberflächen geeignet, wobei durch gleichzeitig durch aus der Reinigungsvorrichtung austretende Reinigungsflüssigkeitsstrahlen die mechanisch abgelösten Verunreinigungen entfernt werden können. Die Reinigungsflüssigkeit kann mit einem üblichen Jet-Lavage-System verbunden werden, wodurch eine Reinigungsflüssigkeit in die Reinigungsvorrichtung gepumpt werden kann. Die Reinigungsflüssigkeit kann bevorzugt gepulst aus der Reinigungsvorrichtung austreten.

### TECHNISCHER HINTERGRUND

In der septischen Knochenchirurgie ist die Reinigung von debridierten Knochen- und Weichgewebearealen bei der Revision von infizierten Gelenkendoprothesen und die Reinigung von infizierten Osteosyntheseplatten durch Lavagierung bekannt. Dafür werden medizinische Spülvorrichtungen, sogenannte Lavage-Systeme, eingesetzt. Diese Lavage-Systeme werden hierin auch als "Lavagevorrichtung" bezeichnet. Als Reinigungsflüssigkeiten sind physiologische Kochsalzlösung, Ringer-Lösung und Ringer-Lactat-Lösung bekannt. Das Ziel der Lavagierung besteht in der Entfernung von Resten des infizierten Gewebes, von Biofilmresten, von Blut und von Wundsekret. Lavage-Systeme sind seit langem bekannt. Exemplarisch sind dafür Vorrichtungen gemäß den Patentschriften EP3187208, US4583531, US5779702, US6059754 und US2019151531. Problematisch ist jedoch, dass mit den üblichen Jet-Lavage-Systemen festhaftende Biofilme und besonders Biofilme in Hinterschnittbereichen nur bedingt oder auch gar nicht abgelöst werden können.

In der septischen Chirurgie nehmen die sogenannten DAIR-Prozeduren (Debridement, antibiotics and implant retention) zur Behandlung von infizierten Kniegelenkendoprothesen, Hüftgelenk Endoprothesen und Schultergelenkendoprothesen zu. Das Ziel dieser OP-Technik ist es, die Infektion der Gelenkendoprothese und des umgebenden Gewebes nach Eröffnung des Gelenks durch Debridement und anschließende lokale Applikation von Antibiotika so zu bekämpfen, dass das künstliche Gelenk erhalten bleibt. Die kostenintensive und für den Patienten belastende Implantation einer Revisionsgelenkendoprothese soll vermieden werden.

Diese Prozeduren werden insbesondere bei früh infizierten künstlichen Gelenkendoprothesen angewendet, Dabei werden zuerst bewegliche Implantatkomponenten, wie PE-Inlays, aus dem künstlichen Gelenk entfernt. Infiziertes Gewebe wird debridiert und Biofilme, die an den Metalloberflächen der Gelenkendoprothese anhaften, versucht man durch Bürsten und Lavagieren möglichst vollständig zu entfernen. Danach werden neue bewegliche Gelenkkomponenten, wie PE-Inlays, in die gereinigte Gelenkendoprothese eingesetzt. Problematisch ist dabei die Entfernung der Biofilme an den Metalloberflächen, insbesondere an den Kondylen von Kniegelenken. Biofilme können extrem klebrig und anhaftend sein. Das bedeutet, dass trotz Bürsten und anschließendem Lavagieren Biofilme oder Biofilmreste an den Metalloberflächen verbleiben können. Diese Biofilmreste enthalten lebende Mikroorganismen und können zu einer Reinfektion der Gelenkendoprothese und des umgebenden Gewebes führen.

### BEVORZUGTE AUSFÜHRUNGSFORMEN

Die Aufgabe der vorliegenden Erfindung besteht darin, eines oder mehrere der oben geschilderten und weitere Probleme des Stands der Technik zu lösen. Eine Aufgabe der Erfindung ist es, Biofilme durch Bürsten der Implantatoberfläche abzutragen und zeitgleich die abgelösten Biofilmreste durch eine Reinigungsflüssigkeit von der Implantatoberfläche zu entfernen. Dadurch können die klebrigen Biofilme nach der mechanischen Ablösung nicht wieder auf der Implantatoberfläche anhaften. Eine weitere Aufgabe der Erfindung ist es, die mit der Reinigungsflüssigkeit vermischten Biofilmreste sofort aus dem Gelenksitus zu entfernen, so dass eine Ablagerung der abgelösten Biofilmreste auf dem umgebenden Gewebe, der Gelenkkapsel, wirksam verhindert werden kann.

Die Vorrichtung kann bevorzugt ein manuelles Bürsten, auch in Hinterschnitten, ermöglichen und gleichzeitig können die durch Büsten abgelösten Verunreinigungen, wie Biofilmreste, sofort durch Spülflüssigkeitsstrahlen entfernt werden, so dass die Verunreinigungen sich nicht an die gereinigten Oberflächen anlagern können. Die Vorrichtung selbst kann bevorzugt keine eigene aktive Antriebsvorrichtung und auch keine Pumpe enthalten. Die Vorrichtung kann bevorzugt möglichst einfach und kostengünstig aus Kunststoffteilen zu fertigen sein. Weiterhin kann die Vorrichtung bevorzugt durch Ethylenoxid oder Gammasterilisation sterilisierbar sein.

Einige oder alle Aufgaben werden gelöst durch hierin beschriebene Verfahren und Vorrichtungen, insbesondere denjenigen, die in den Patentansprüchen beschrieben sind. Bevorzugte Ausführungsformen der Erfindung werden nachstehend beschrieben.

Eine erste Ausführungsform beschreibt eine Vorrichtung zur chirurgischen Reinigung infizierter Implantate, aufweisend
einen Bürstenkopf, welcher einen Borstenbereich mit einer Vielzahl von Borsten aufweist;
ein Zuleitungselement, das zum Transport einer Reinigungsflüssigkeit zum Bürstenkopf eingerichtet ist;
ein Ableitungselement, das zur Entfernung von Flüssigkeit vom Bürstenkopf eingerichtet ist; und ein Verbindungselement, welches zur lösbaren Verbindung des Zuleitungselements und des Ableitungselements mit einer externen Lavagevorrichtung eingerichtet ist.

Eine zweite Ausführungsform beschreibt eine Vorrichtung gemäß der ersten Ausführungsform, wobei die Vorrichtung eingerichtet ist, ein Verhältnis der Fördermengen von Ableitungselement zu Zuleitungselement von mindestens 1 zu erreichen.

Eine dritte Ausführungsform beschreibt eine Vorrichtung gemäß der ersten oder zweiten Ausführungsform, wobei der Bürstenkopf eingerichtet ist, eine oszillierende Kraft auf die Borsten auszuüben.

Eine vierte Ausführungsform beschreibt eine Vorrichtung nach einer der vorangehenden Ausführungsformen, wobei die Vorrichtung ein Antriebselement zur Erzeugung der oszillierenden Kraft aufweist, wobei das Antriebselement bevorzugt zum Antrieb durch die Strömung der Reinigungsflüssigkeit durch das Zuleitungselement eingerichtet ist.

Eine fünfte Ausführungsform beschreibt eine Vorrichtung nach einer der vorangehenden Ausführungsformen, weiterhin umfassend ein Ventil, das zwischen dem Verbindungselement und dem Bürstenkopf angeordnet ist, und eingerichtet ist, den Fluss einer Reinigungsflüssigkeit durch das Zuleitungselement zu steuern.

Eine sechste Ausführungsform beschreibt eine Vorrichtung nach einer der vorangehenden Ausführungsformen, wobei der Bürstenkopf Borsten unterschiedlicher Länge aufweist, um die Reinigung von Hinterschneidungen zu erleichtern, wobei die Borsten bevorzugt bogenförmig in dem Borstenbereich angeordnet sind.

Eine siebte Ausführungsform beschreibt eine Vorrichtung nach einer der vorangehenden Ausführungsformen, wobei das Zuleitungselement eine Austrittsöffnung aufweist, die innerhalb des Borstenbereichs angeordnet ist.

Eine achte Ausführungsform beschreibt eine Vorrichtung nach einer der vorangehenden Ausführungsformen, wobei das Ableitungselement eine Ansaugöffnung aufweist, die am proximalen Ende des Borstenbereichs angeordnet ist.

Eine neunte Ausführungsform beschreibt eine Vorrichtung nach einer der vorangehenden Ausführungsformen, wobei die Vorrichtung eingerichtet ist, Reinigungsflüssigkeit aus dem Zuleitungselement durch die Borsten zu leiten und an der Spitze der Borsten freizusetzen.

Eine zehnte Ausführungsform beschreibt eine Vorrichtung nach einer der vorangehenden Ausführungsformen, wobei die Vorrichtung keine Pumpe aufweist.

Eine elfte Ausführungsform beschreibt eine Vorrichtung nach einer der vorangehenden Ausführungsformen, wobei das Zuleitungselement, und bevorzugt auch das Ableitungselement, durch das Verbindungselement hindurchgeführt sind.

Eine zwölfte Ausführungsform beschreibt eine Vorrichtung nach einer der vorangehenden Ausführungsformen, weiterhin umfassend ein
Griffelement, welches zwischen dem Verbindungselement und dem Borstenelement angeordnet ist, wobei das Griffelement zur manuellen Führung der Vorrichtung durch einen Benutzer eingerichtet ist.

Eine dreizehnte Ausführungsform beschreibt eine Vorrichtung gemäß der zwölften Ausführungsform, weiterhin umfassend ein Stielelement, welches das Griffelement mit dem Bürstenkopf verbindet.
eine vierzehnte Ausführungsform beschreibt eine Vorrichtung gemäß der dreizehnten Ausführungsform, wobei das Stielelement eine Länge von 5 bis 10 cm aufweist und/odereinen Durchmesser von höchstens 10 mm aufweist.

Eine fünfzehnte Ausführungsform beschreibt eine Vorrichtung gemäß einer der vorangehenden Ausführungsformen, wobei das Zuleitungselement einen flexiblen Schlauch umfasst und/oder eine Länge von mindestens 80 cm aufweist.

### AUSFÜHRLICHE BESCHREIBUNG

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse und Vorrichtungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Ein erster Aspekt der Erfindung betrifft eine Vorrichtung zur chirurgischen Reinigung infizierter Implantate, welche
einen Bürstenkopf, welcher einen Borstenbereich mit einer Vielzahl von Borsten aufweist;
ein Zuleitungselement, das zum Transport einer Reinigungsflüssigkeit zum Bürstenkopf eingerichtet ist;
ein Ableitungselement, das zur Entfernung von Flüssigkeit vom Bürstenkopf eingerichtet ist; und
ein Verbindungselement, welches zur lösbaren Verbindung des Zuleitungselements und des Ableitungselements mit einer externen Lavagevorrichtung eingerichtet ist, aufweist.

Die Vorrichtung ist insbesondere zur Reinigung bakteriell infizierten Gewebes vorgesehen, beispielsweise während eines chirurgischen Eingriffs. Die Vorrichtung kann bevorzugt zur Reinigung infizierter Implantate verwendet werden.

Die Vorrichtung weist einen Bürstenkopf auf, an welchem eine Vielzahl von Borsten in einem Borstenbereich angeordnet sind. Die Borsten sind bevorzugt aus einem biokompatiblen Polymer gebildet, zum Beispiel Polyethylen, Polypropylen, Polyamid oder ähnliches.

Die Vorrichtung weist ein Zuleitungselement auf, welches zum Transport einer Reinigungsflüssigkeit eingerichtet ist. Insbesondere kann mithilfe des Zuleitungselements eine Reinigungsflüssigkeit von einem Verbindungselement zum Bürstenkopf geleitet werden, um im Bereich des Bürstenkopfs freigesetzt zu werden.

Die Vorrichtung weist ein Ableitungselement auf, welches zur Aufnahme und zum Transport von Flüssigkeit eingerichtet ist. Das Ableitungselement kann verwendet werden, um Reinigungsflüssigkeit, die zunächst über das Zuleitungselement am Bürstenkopf freigesetzt wurde, im benutzten bzw. verunreinigten Zustand zu entfernen. Hierdurch können infiziertes Gewebe und Biofilme aus einem Patienten entfernt werden, sodass nicht die Gefahr besteht, dass Krankheitserreger im Patienten verbleiben oder sogar verteilt werden. Insbesondere kann ein Wiederanhaften von Bakterien aus Biofilmen an ein Implantat vermieden werden.

Die hierin beschriebenen Vorrichtungen sind insbesondere zum Betrieb mit einer externen Lavagevorrichtung geeignet. Beispiele für Lavagevorrichtungen, mit denen die hierin beschriebenen Vorrichtungen betrieben werden können, sind beispielsweise in EP2662146A2, US4,583,531A, US4,278,078A und US5,542,918A beschrieben. Solche Lavagevorrichtungen können eine Pumpe umfassen, die beispielsweise mit einem Elektromotor oder einem Druckluftmotor ausgestattet sein kann. Bevorzugt können die hierin beschriebenen Vorrichtungen mit Lavagevorrichtungen betrieben werden, welche gepulste Sprühstöße erzeugen können. Die Lavagevorrichtungen können beispielsweise Pumpen umfassen, wie sie in EP2619415A1, EP2910270A1 oder EP2873856A1 beschrieben sind.

Zum Anschluss an eine solche Lavagevorrichtung ist die hierin beschriebene Vorrichtung mit einem Verbindungselement ausgestattet. Das Verbindungselement kann formschlüssig oder kraftschlüssig mit einer externen Lavagevorrichtung verbindbar sein. Beispielsweise kann das Verbindungselement ein Rastelement aufweisen, um die Vorrichtung mit einer Lavagevorrichtung zu verbinden. Das Verbindungselement kann insbesondere eingerichtet sein, das Zuleitungselement und das Ableitungselement flüssigkeitsleitend mit einer Lavagevorrichtung zu verbinden. Das Verbindungselement kann insbesondere eine Öffnung umfassen, die zur Abgabe von Flüssigkeit aus dem Ableitungselement an eine Lavagevorrichtung eingerichtet ist. Das Verbindungselement kann weiterhin eine Öffnung umfassen, die zur Aufnahme einer Reinigungsflüssigkeit aus einer Lavagevorrichtung eingerichtet ist, um die Reinigungsflüssigkeit in das Zuleitungselement zu leiten.

Aus diesem Grund benötigen die hierin beschriebenen Vorrichtungen selbst keine Pumpe. Demnach weisen die hierin beschriebenen Vorrichtungen bevorzugt keine eigene Pumpe auf. Eine Pumpe ist eine Vorrichtung, welche den Transport einer Flüssigkeit durch aktive Veränderung des Drucks der zu transportierenden Flüssigkeit bewirken kann.

In einigen Ausführungsformen ist die Vorrichtung eingerichtet, ein Verhältnis der Fördermengen von Ableitungselement zu Zuleitungselement von mindestens 1 zu erreichen. Dies bedeutet, dass die Vorrichtung eingerichtet ist, gleichzeitig mindestens genauso viel Flüssigkeit über das Ableitungselement abzuführen, wie über das Zuleitungselement am Bürstenkopf freigesetzt wird. Hierdurch kann besser sichergestellt werden, dass gelöste Biofilme und abgelöstes infiziertes Gewebe wirkungsvoll aus dem Patienten entfernt werden können. In einigen Ausführungsformen beträgt das Verhältnis der Fördermengen von Ableitungselement zu Zuleitungselement mindestens 1,0; 1,2; 1,5; oder mindestens 2. Dies kann beispielsweise dadurch erreicht werden, dass der Innendurchmesser des Ableitungselement im Vergleich zum Innendurchmesser des Zuleitungselements entsprechend größer dimensioniert ist.

In einigen Ausführungsformen ist der Bürstenkopf eingerichtet, eine oszillierende Kraft auf die Borsten auszuüben. Beispielsweise kann der Bürstenkopf beweglich sein, zum Beispiel aufgrund elastischer Materialeigenschaften der Vorrichtung, oder durch Einbau von Gelenken. Hierdurch können die Borsten in Schwingung versetzt werden. Dadurch kann eine bessere Abtragung infizierten Gewebes oder von Biofilmen erreicht werden.

In einigen Ausführungsformen umfasst die Vorrichtung ein Antriebselement zur Erzeugung einer solchen oszillierenden Kraft. Hierbei kann es sich entweder um ein aktives Antriebselement handeln, zum Beispiel ein Elektromotor, oder es kann ein passives Antriebselement verwendet werden. Ein Beispiel für ein passives Antriebselement ist ein Vibrationsmotor, der durch die Strömung einer Reinigungsflüssigkeit durch das Zuleitungselement betrieben wird. Beispielsweise kann hierzu ein durch einen Flüssigkeitsstrom angetriebenes Flügelrad verwendet werden, das eine nichtsymmetrische Masseverteilung hat. Weitere Beispiele für Antriebselemente sind Kugelvibrationsmotoren und Walzen-Vibrationsmotoren. Durch eine kreisförmige Bewegung eines kugelförmigen Massekörpers kann eine Unwucht erzeugt werden, die zu Vibrationen des Stielelements und/oder des Bürstenkopfes führen, wodurch die Reinigungswirkung verstärkt werden kann.

Die oszillierende Kraft kann auch durch Sprühstöße einer Reinigungsflüssigkeit erzeugt werden. Hierzu kann die Vorrichtung an eine Lavagevorrichtung angeschlossen werden, welche die gepulste Abgabe einer Reinigungsflüssigkeit ermöglicht. Die externe Lavagevorrichtung kann dazu mit einer Pumpe und einer Steuerung ausgestattet sein, welche einen solchen Pulsbetrieb ermöglicht.

In einigen Ausführungsformen umfasst die Vorrichtung ein Ventil, das eingerichtet ist, den Fluss einer Reinigungsflüssigkeit durch das Zuleitungselement zu steuern. Das Ventil kann bevorzugt zwischen dem Verbindungselement und dem Bürstenkopf angeordnet sein.

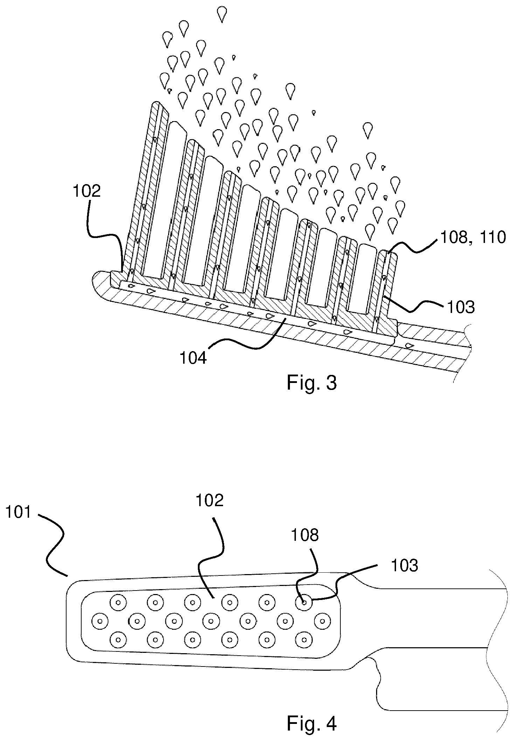

Der Bürstenkopf kann Borsten mit unterschiedlicher Länge aufweisen, um die Reinigung von Hinterschneidungen zu erleichtern. Beispielsweise können die unterschiedlich langen Borsten bogenförmig auf dem Bürstenkopf angeordnet sein, wie beispielsweise in Figur 3 gezeigt. Zum Beispiel können die Borsten im distalen Bereich des Bürstenkopfs länger sein als die Borsten am proximalen Bereich des Bürstenkopfs. Der distale Bereich des Bürstenkopfs ist diejenige Seite, die am weitesten vom Verbindungselement entfernt angeordnet ist. Der proximale Bereich des Bürstenkopfs ist diejenige Seite, die in Richtung des Griffelements (sofern vorhanden) und des Verbindungselements weist.

In einigen Ausführungsformen weist das Zuleitungselement eine Austrittsöffnung auf, welche innerhalb des Borstenbereichs angeordnet ist. Bevorzugt weist die Vorrichtung eine Vielzahl von Austrittsöffnungen auf. Die Austrittsöffnungen können am Bürstenkopf in der Nähe der Borsten, bevorzugt innerhalb des Borstenbereichs, oder an den Borsten selbst angeordnet sein, oder beides. Die Borsten können hohl sein, um den Transport einer Reinigungsflüssigkeit zu ermöglichen. Die Vorrichtung kann eingerichtet sein, Reinigungsflüssigkeit aus dem Zuleitungselement durch die Borsten zu leiten, und an der Spitze der Borsten freizusetzen. Das Zuleitungselement und/oder das Ableitungselement sind bevorzugt durch das Verbindungselement hindurchgeführt. Sie verlaufen bevorzugt innerhalb des Verbindungselements und sind dadurch besser vor Beschädigung oder Störung geschützt.

Die Vorrichtung kann ein Griffelement aufweisen, welches zur manuellen Führung der Vorrichtung durch einen Benutzer eingerichtet ist. Das Griffelement kann zwischen dem Verbindungselement und dem Bürstenkopf angeordnet sein. Mithilfe des Griffelements kann ein Benutzer den Bürstenkopf sicher greifen und halten, und während der Verwendung in geeigneter Weise manuell positionieren und führen.

Die Vorrichtung kann weiterhin ein Stielelement aufweisen, welches das Griffelement mit dem Bürstenkopf verbindet. Demnach ist das Stielelement zwischen dem Griffelement und dem Bürstenkopf angeordnet. Das Stielelement kann bevorzugt flexibel sein, um eine Bewegung des Bürstenkopfs zu ermöglichen, wie oben ausführlicher dargestellt. Das Stielelement kann beispielsweise eine Länge von fünf bis 10 cm aufweisen. Das Stielelement kann einen Durchmesser von 10 mm oder weniger aufweisen, beispielsweise weniger als 9 mm oder weniger als 7 mm. Dies kann es dem Benutzer erleichtern, schwer zugängliche Körperregionen besser zu erreichen, zum Beispiel bei DAIR-Prozeduren an infizierten Kniegelenkendoprothesen. Bevorzugt weist das Stielelement einen höheren Außendurchmesser auf als das Ableitungselement.

Bevorzugt sind Stielelement und Ableitungselement parallel zueinander angeordnet. Bevorzugt sind Stielelement und Ableitungselement direkt miteinander verbunden. Bevorzugt sind Stielelement und Ableitungselement biegsam.

In einigen Ausführungsformen ist die Vorrichtung teilweise oder vollständig aus Kunststoff gefertigt. Insbesondere können Bürstenkopf, Stielelement, Griffelement und/oder Verbindungselement jeweils einen Kunststoff umfassen, oder vollständig aus Kunststoff bestehen.

In einigen Ausführungsformen ist die Vorrichtung zur Sterilisierung durch Ethylenoxid oder Gammasterilisation eingerichtet, d.h. sie kann ohne Funktionsbeeinträchtigung durch die genannten Verfahren sterilisiert werden.

Das Zuleitungselement kann einen flexiblen Schlauch umfassen. Das Zuleitungselement kann eine Länge von mindestens 80 cm aufweisen. In einigen Ausführungsformen beträgt die Länge des Zuleitungselements im Bereich zwischen dem Verbindungselement und dem Griffelement oder dem Bürstenkopf mindestens 80 cm. Dies bietet dem Benutzer eine bessere Beweglichkeit der Vorrichtung, ohne die externe Lavagevorrichtung mitbewegen zu müssen.

In einigen Ausführungsformen umfasst der Bürstenkopf einen gewinkelt angeordneten Ausstromkanal. Der Ausstromkanal kann in Bezug auf die Ausrichtung der Borsten in einem Winkel von mehr als 15°, zum Beispiel ungefähr 45°, angeordnet sein. Hierdurch können Sprühstöße der Reinigungsflüssigkeit in den Bereich neben dem Bürstenkopf abgegeben werden, um die Reinigungsleistung der Bürste zu verbessern. Die Reinigungsflüssigkeit kann am äußeren Ende des Ausstromkanal über eine Ausströmöffnung abgegeben werden.

Ein weiterer Aspekt betrifft ein Therapieverfahren, in welchem eine hierin beschriebene Vorrichtung zur Entfernung von Biofilmen oder infiziertem Gewebe eingesetzt wird. Hierbei werden Biofilme oder infiziertes Gewebe mithilfe der Borsten und einer über das Zuleitungselement freigesetzten Reinigungsflüssigkeit entfernt, wobei die Reinigungsflüssigkeit gemeinsam mit dem zu entfernenden Material über das Ableitungselement aus dem Patienten verbracht werden. Die Vorrichtung kann dabei mit einer externen Lavagevorrichtung verbunden sein. In einer Ausführungsform werden dabei pulsierende Sprühstöße der Reinigungsflüssigkeit über den Bürstenkopf abgegeben. In einer Ausführungsform wird über die Borsten eine oszillierende Kraft ausgeübt, wie hierin beschrieben.

Die Reinigungsflüssigkeit kann ein Detergens, eine Puffersubstanz und/oder einen antibakteriellen Wirkstoff umfassen. Die Reinigungsflüssigkeit kann eine physiologische Kochsalzlösung, Ringer-Lösung oder Ringer-Lactat-Lösung umfassen.

Die Vorrichtung kann für ein Debridement und insbesondere für DAIR-Prozeduren eingesetzt werden. Die Vorrichtung kann beispielsweise im Rahmen einer Knie- oder Hüftgelenksoperation eingesetzt werden, insbesondere bei einer Revisions-OP.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

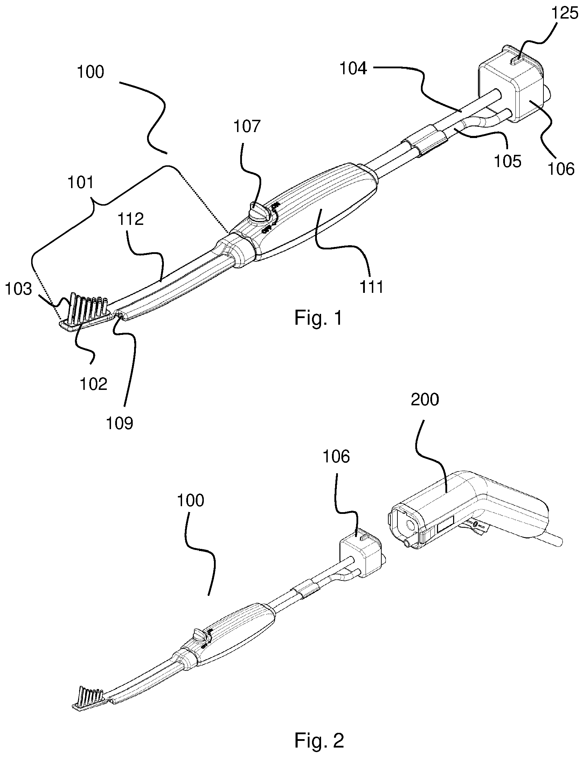

**Figur 1** zeigt beispielhaft eine Ausführungsform einer erfindungsgemäßen Vorrichtung 100. Die Vorrichtung umfasst einen Bürstenkopf 101. Der Bürstenkopf 101 umfasst ein Stielelement 112 mit einem Borstenbereich 102. In dem Borstenbereich 102 sind büschelweise eine Vielzahl von Borsten 103 angeordnet. Das Stielelement 112 umfasst angrenzend an den Borstenbereich 102 eine Ansaugöffnung 109. Die Ansaugöffnung 109 kann zur Aufnahme und Entfernung von Reinigungsflüssigkeit dienen, welche durch die Verwendung mit der erfindungsgemäßen Vorrichtung infiziertes Gewebe oder Biofilme abgetragen hat. Der Bürstenkopf 101 ist lösbar mit einem Griffelement 111 verbunden. Das Griffelement 111 umfasst weiterhin ein Ventil zur Steuerung eines Zuleitungselements 104. Das Zuleitungselement 104 ist innerhalb des Griffelements 111 und des Bürstenkopfs 101 geführt. Das Zuleitungselement 104 dient der Zuleitung einer Reinigungsflüssigkeit in den Bürstenkopf 101. Weiterhin umfasst die Vorrichtung ein Ableitungselement 105, welches zur Ableitung der durch die Ansaugöffnung 109 aufgenommenen Reinigungsflüssigkeit dienen kann. Das Zuleitungselement 104 und das Ableitungselement 105 sind jeweils zu einem Verbindungselement 106 geführt, welches zum Anschluss an eine externe Lavagevorrichtung vorgesehen ist. Zu diesem Zweck umfasst das Verbindungselement 106 ein Rastelement 125, welches mit einer Lavagevorrichtung verbunden werden kann.
**Figur 2** zeigt die Verbindung einer erfindungsgemäßen Vorrichtung 100 mit einer externen Lavagevorrichtung 200. Hierbei wird das Verbindungselement 106 der Vorrichtung 100 formschlüssig mit einer Lavagevorrichtung 200 verbunden, wobei das Zuleitungselement 104 und das Ableitungselement 105 jeweils mit entsprechenden Anschlüssen der Lavagevorrichtung 200 flüssigkeitsdicht verbunden werden.
**Figur 3** zeigt einen Ausschnitt des Bürstenkopfs einer erfindungsgemäßen Vorrichtung in einer Querschnittsansicht. Das Zuleitungselement 104 ist eingerichtet, Reinigungsflüssigkeit zum Borstenbereich 102 zu leiten. Im hier gezeigten Beispiel umfasst der Borstenbereich 102 hohlförmige Borsten 103, die flüssigkeitsleitend mit dem Zuleitungselement 104 verbunden sind. Somit kann Reinigungsflüssigkeit durch die hohlen Borsten 103 zu einer Austrittsöffnung 108 an der Spitze 110 der Borsten geleitet werden, wo die Reinigungsflüssigkeit aus der Austrittsöffnung 108 freigesetzt werden kann. Die aus den Austrittsöffnungen freigesetzte Reinigungsflüssigkeit ist hier symbolisch in Form von Tropfen dargestellt. Die Borsten 103 weisen in diesem Beispiel eine unterschiedliche Länge auf. Die Borsten 103 weisen am distalen Ende des Bürstenkopfs (in Figur 3 links gezeigt) eine höhere Länge auf als am proximalen Ende des Bürstenkopfs (in Figur 3 rechts gezeigt), sodass sich in Bezug auf die Spitzen der Borsten eine insgesamt bogenförmige Anordnung ergibt. Eine solche Geometrie kann vorteilhaft sein, um Biofilme und bakteriell belastetes Gewebe im Bereich von schwer zugänglichen Körperregionen zu entfernen, insbesondere im Bereich von Hinterschneidungen. Ein Beispiel hierfür sind DAIR-Prozeduren an infizierten Kniegelenkendoprothesen, wobei eine Reinigung insbesondere auch im Spalt zwischen der Femur- und der Tibiakomponente des Gelenks erforderlich ist.
**Figur 4** zeigt den in Figur 3 gezeigten Ausschnitt eines Bürstenkopfs 101 in einer Aufsicht. Innerhalb des Borstenbereichs 102 sind eine Vielzahl von Borsten 103 angeordnet, die jeweils einen Hohlraum umfassen, der eine Reinigungsflüssigkeit aus dem Zuleitungselement 104 zu einer Austrittsöffnung 108 an der Spitze der Borsten leiten kann.

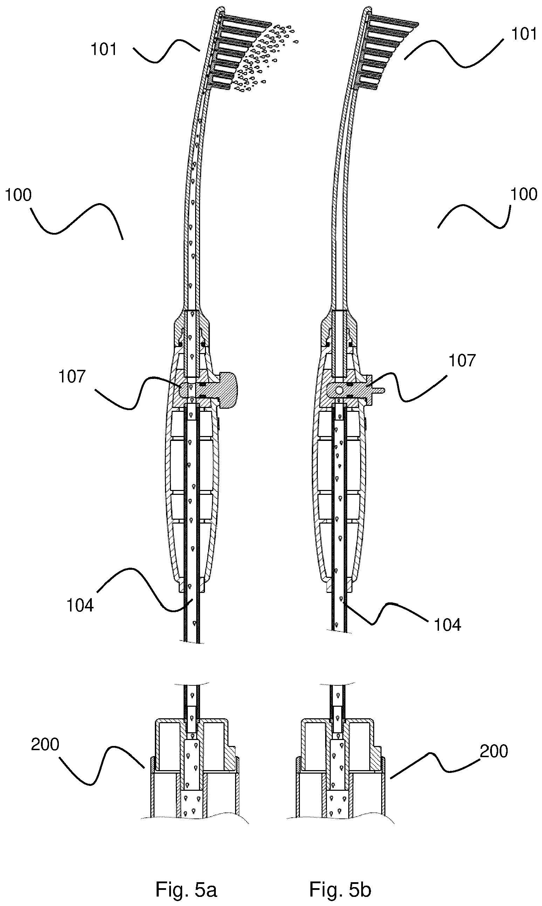

**Figur 5a** zeigt in einer Querschnittsansicht eine Ausführungsform einer erfindungsgemäßen Vorrichtung 100, welche mit einem Ventil 107 ausgestattet ist. Das Ventil ist dazu eingerichtet, den Fluss einer Reinigungsflüssigkeit durch das Zuleitungselement 104 zu steuern. In Figur 5a ist das Ventil 107 in einer geöffneten Konfiguration gezeigt, welche einen freien Durchfluss einer Reinigungsflüssigkeit aus einer externen Lavagevorrichtung 200 durch das Zuleitungselement 104 zum Bürstenkopf 101 erlaubt. Somit kann die Reinigungsflüssigkeit im Bereich des Bürstenkopfs 101 freigesetzt werden.
**Figur 5b** zeigt die in Figur 5a dargestellte Vorrichtung mit einem Ventil 107 in geschlossener Konfiguration. Die Reinigungsflüssigkeit, die aus der Lavagevorrichtung 200 in die Vorrichtung 100 gepumpt wird, kann über das Zuleitungselement 104 nur bis zum geschlossenen Ventil 107 geführt werden, und nicht bis in den Bereich des Bürstenkopfs 101 gelangen. Daher erfolgt bei geschlossenem Ventil 107 keine Freisetzung der Reinigungsflüssigkeit am Bürstenkopf 101.

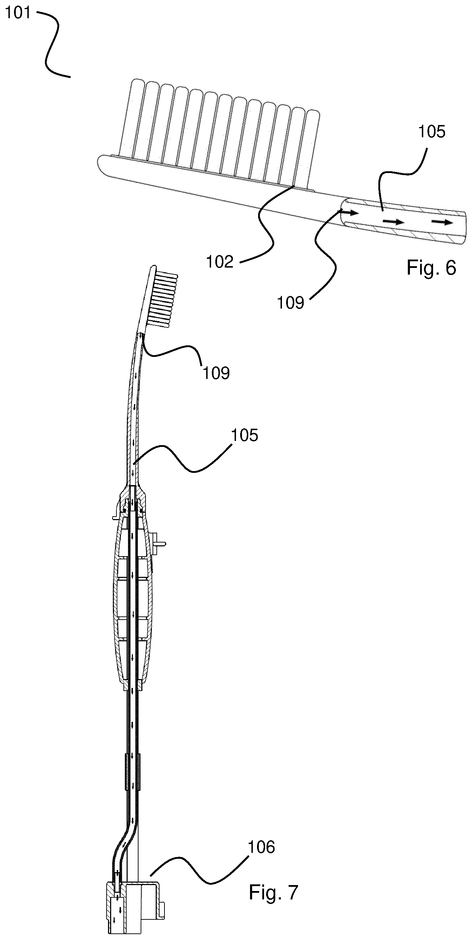

**Figur 6** zeigt eine Querschnittansicht eines Bürstenkopfs 101 einer erfindungsgemäßen Vorrichtung. In diesem Beispiel weisen alle Borsten im Wesentlichen dieselbe Länge auf. Angrenzend an den Borstenbereich 102 ist eine Ansaugöffnung 109 angeordnet. Diese erlaubt die Aufnahme und Ableitung von benutzter Reinigungsflüssigkeit und zu entfernenden Verunreinigungen über das Ableitungselement 105. Die Strömungsrichtung der zu transportierenden Flüssigkeit ist mit Pfeilen gekennzeichnet.
**Figur 7** zeigt eine Querschnittansicht einer erfindungsgemäßen Vorrichtung 100. Die Strömungsrichtung der zu transportierenden Flüssigkeit ist mit Pfeilen gekennzeichnet. Diese gelangt durch das Ableitungselement 105 von der Ansaugöffnung 109 in Richtung des Verbindungselements 106, wo sie von einer externen Lavagevorrichtung aufgenommen werden kann.

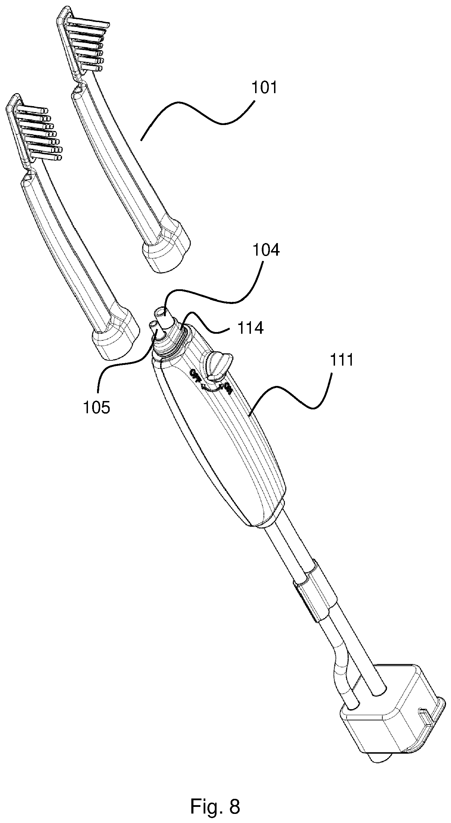

**Figur 8** zeigt eine Ausführungsform einer erfindungsgemäßen Vorrichtung mit abnehmbaren Bürstenkopf 101. Der Bürstenkopf 101 kann lösbar mit dem Griffelement 111 der Vorrichtung verbunden werden, indem er auf eine Tülle 114 aufgesteckt wird. Das Zuleitungselement 104 und das Ableitungselement 105 sind dabei durch die Tülle 114 hindurchgeführt, und ragen an deren Ende über diese hinaus, um eine formschlüssige Verbindung mit dem Bürstenkopf 101 zu ermöglichen.

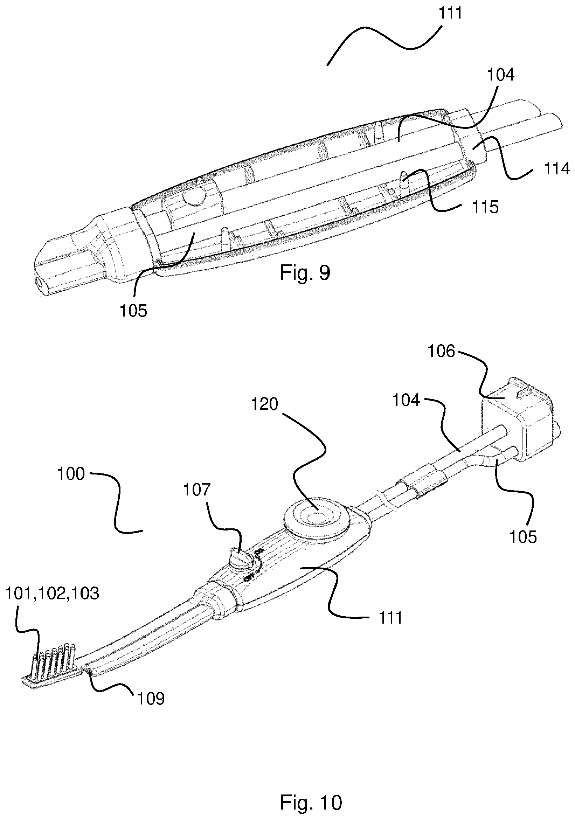

**Figur 9** zeigt eine isometrische Querschnittansicht eines Griffelements 111. Das Griffelement ist in diesem Beispiel aus zwei Halbschalen gebildet, die über Rastelemente 115 miteinander verbunden sind. Das Zuleitungselement 104 und das Ableitungselement 105 sind durch den Innenraum des Griffelements 111 und eine Tülle 114 an der Gehäuseöffnung des Griffelements hindurchgeführt.
**Figur 10** zeigt eine Ausführungsform einer erfindungsgemäßen Vorrichtung, die mit einem Antriebselement 120 ausgestattet ist. Das Antriebselement 120 ist dazu eingerichtet, eine oszillierende Kraft zu erzeugen, die auf den Bürstenkopf 101 und insbesondere die Borsten 103 übertragen werden kann, um die Reinigungsleistung der Vorrichtung zu erhöhen. Das Antriebselement 120 und ein Ventil 107 sind am Griffelement 111 angeordnet. Die Vorrichtung kann über ein Verbindungselement 106 an eine externe Lavagevorrichtung angeschlossen werden, wobei das Zuleitungselement 104 und das Ableitungselement 105 flüssigleitend mit der Lavagevorrichtung verbunden werden.

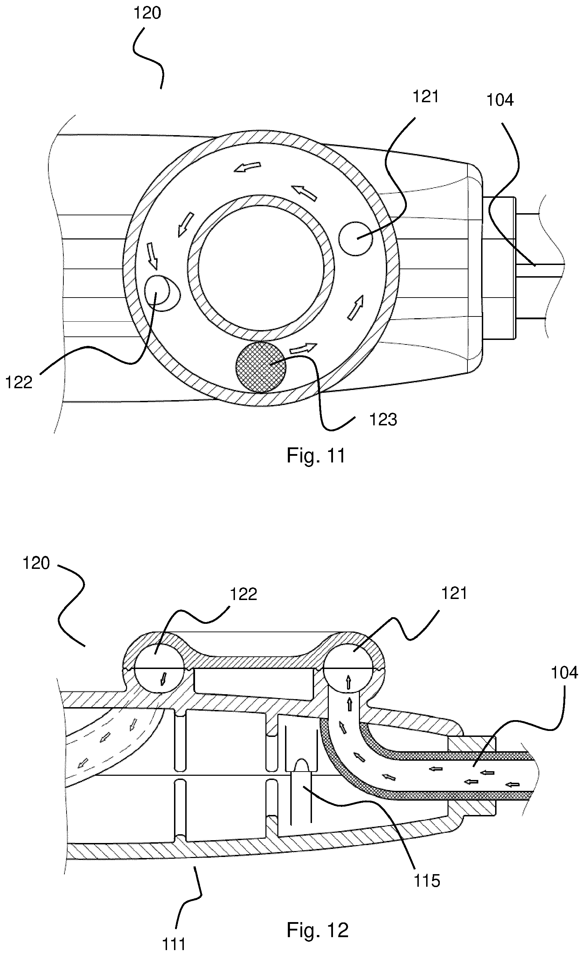

**Figur 11** zeigt einen Querschnitt durch ein Antriebselement 120 als Aufsicht. Das Antriebselement ist in diesem Beispiel ein passives Antriebselement, welches keine eigene Energieversorgung benötigt, sondern durch die hindurchfließende Reinigungsflüssigkeit angetrieben werden kann. Hierzu wird das Antriebselement 120 flüssigleitend an das Zuleitungselement 104 angebunden, sodass die Reinigungsflüssigkeit durch eine Einströmöffnung 121 in das Antriebselement 120 geleitet wird, durch einen Kanal innerhalb des Antriebselement fließt, hierdurch eine Kugel 123 auf einer Kreisbahn bewegt, und durch eine Ausströmöffnung 122 wieder in das Zuleitungselement 104 gelangt. Die Fließrichtung der Reinigungsflüssigkeit ist durch Pfeile gekennzeichnet. Dieses Funktionsprinzip findet in ähnlicher Weise in pneumatischen Kugel-Vibrationsmotoren Anwendung.
**Figur 12** zeigt einen Querschnitt durch ein Antriebselement 120 als Seitenansicht. Eine Reinigungsflüssigkeit fließt durch das Zuleitungselement 104 von einer externen Quelle in die Einströmöffnung 121 des Antriebselements 120, und fließt über die Ausströmöffnung 122 weiter in Richtung des Bürstenkopfs der Vorrichtung. Die beiden Halbschalen des Griffelements 111 sind durch Rastelemente 115 zusammengehalten.

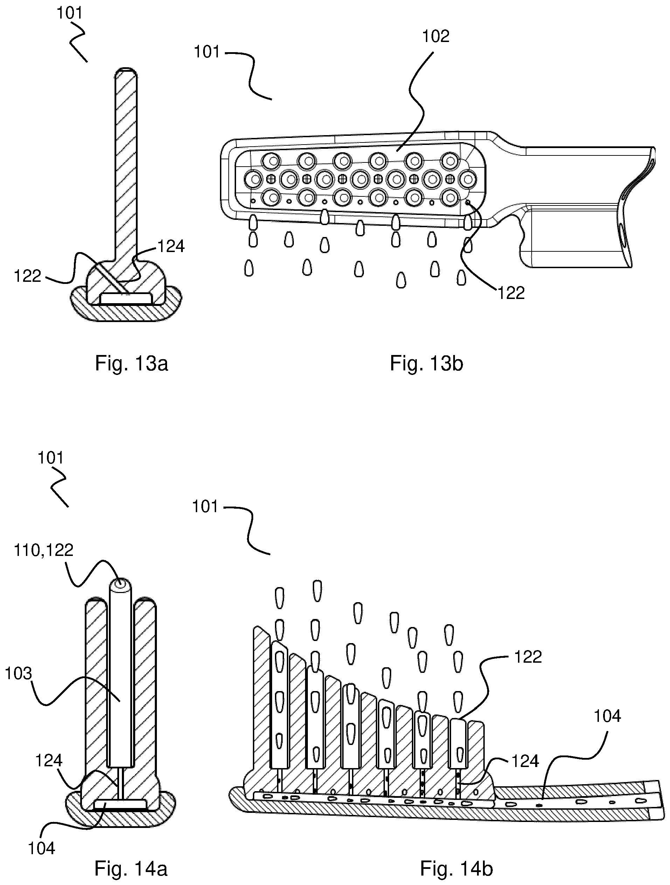

**Figur 13a** zeigt eine Querschnittansicht eines Bürstenkopfs 101, der über einen gewinkelt angeordneten Ausstromkanal 124 verfügt. Der Ausstromkanal 124 ist in Bezug auf die Ausrichtung der Borsten in einem Winkel von mehr als 15°, in diesem Beispiel ungefähr 45°, angeordnet. Hierdurch können Sprühstöße der Reinigungsflüssigkeit in den Bereich neben dem Bürstenkopf abgegeben werden, um die Reinigungsleistung der Bürste zu verbessern. Die Reinigungsflüssigkeit kann am äußeren Ende des Ausstromkanal 124 über eine Ausströmöffnung 122 abgegeben werden.
**Figur 13b** zeigt den in Figur 13a dargestellten Bürstenkopf in einer Aufsicht. Die Ausströmöffnungen sind entlang eines Seitenrandes des Bürstenkopfs im Borstenbereich 102 angeordnet.
**Figur 14a** zeigt eine Querschnittansicht eines Bürstenkopfs 101, der über einen angeordneten Ausstromkanal 124 verfügt, welcher das Zuleitungselement 104 flüssigkeitsleitend mit Borsten 103 verbindet, welche einen flüssigkeitsleitenden Hohlraum aufweisen, sodass eine Reinigungsflüssigkeit an eine Ausströmöffnung 122 an der Spitze 110 der Borsten 103 freigesetzt werden kann.
**Figur 14b** zeigt den in Figur 14a dargestellten Bürstenkopf in einer Aufsicht.

Die in Figur 13 und in Figur 14 gezeigten Varianten können alternativ oder in Kombination in der erfindungsgemäßen Vorrichtung Verwendung finden.

### ANWENDUNGSBEISPIEL 1

Die Vorrichtung kann während eines chirurgischen Eingriffs beispielsweise wie folgt zum Debridement verwendet werden, wobei die folgenden Schritte a) bis I) in der angegebenen Reihenfolge ausgeführt werden:
a) Verbinden einer Lavagevorrichtung mit einem Reinigungsflüssigkeitsvorratsbehälter,
b) Verbinden des Adapters der Vorrichtung mit der Lavagevorrichtung,
c) Inbetriebnahme des Motors der Lavagevorrichtung,
d) Pumpen der Reinigungsflüssigkeit durch die Lavagevorrichtung durch das Zuleitungselement zum Bürstenkopf der Vorrichtung,
e) Öffnen des Ventils im Griffelement,
f) Dadurch Einleiten der Spülflüssigkeit entlang des Stielelements in den Bürstenkopf,
g) Manuelles Bürsten der zu reinigenden Oberfläche, wobei zeitgleich die Reinigungsflüssigkeit in Form von Sprühstrahlen aus dem Bürstenkopf bzw. den hohlen Borsten austritt,
h) Wegspülen der abgelösten Verunreinigungen von der zu reinigenden Oberfläche mithilfe der Reinigungsflüssigkeit,
i) Absaugen der gebrauchten Reinigungsflüssigkeit aus Schritt h) durch das Absaugelement und durch das Verbindungselement in die Lavagevorrichtung,
j) Abschalten der Pumpfunktion der Lavagevorrichtung,
k) Trennen des Verbindungselements von der Lavagevorrichtung, und
l) Entsorgen der Vorrichtung.

### LISTE DER BEZUGSZEICHEN

- 100: Vorrichtung
- 101: Bürstenkopf
- 102: Borstenbereich
- 103: Borste
- 104: Zuleitungselement
- 105: Ableitungselement
- 106: Verbindungselement
- 107: Ventil
- 108: Austrittsöffnung
- 109: Ansaugöffnung
- 110: Spitze der Borste
- 111: Griffelement
- 112: Stielelement
- 114: Tülle
- 115: Rastelement (am Griffelement)
- 120: Antriebselement
- 121: Einströmöffnung
- 122: Ausströmöffnung
- 123: Kugel
- 124: Ausstromkanal
- 125: Rastelement (am Verbindungselement)
- 200: Lavagevorrichtung

## Patentansprüche

1. Vorrichtung (100) zur chirurgischen Reinigung infizierter Implantate, aufweisend einen Bürstenkopf (101), welcher einen Borstenbereich (102) mit einer Vielzahl von Borsten (103) aufweist;
ein Zuleitungselement (104), das zum Transport einer Reinigungsflüssigkeit zum Bürstenkopf (101) eingerichtet ist;
ein Ableitungselement (105), das zur Entfernung von Flüssigkeit vom Bürstenkopf (101) eingerichtet ist; und
ein Verbindungselement (106), welches zur lösbaren Verbindung des Zuleitungselements (104) und des Ableitungselements (105) mit einer externen Lavagevorrichtung (200) eingerichtet ist.

2. Vorrichtung nach Anspruch 1, wobei die Vorrichtung eingerichtet ist, ein Verhältnis der Fördermengen von Ableitungselement (105) zu Zuleitungselement (104) von mindestens 1 zu erreichen.

3. Vorrichtung nach Anspruch 1 oder 2 wobei der Bürstenkopf (101) eingerichtet ist, eine oszillierende Kraft auf die Borsten (103) auszuüben.

4. Vorrichtung nach einem der vorangehenden Ansprüche, wobei die Vorrichtung ein Antriebselement (120) zur Erzeugung der oszillierenden Kraft aufweist, wobei das Antriebselement (120) bevorzugt zum Antrieb durch die Strömung der Reinigungsflüssigkeit durch das Zuleitungselement (104) eingerichtet ist.

5. Vorrichtung nach einem der vorangehenden Ansprüche, weiterhin umfassend ein Ventil (107), das zwischen dem Verbindungselement (106) und dem Bürstenkopf (101) angeordnet ist, und eingerichtet ist, den Fluss einer Reinigungsflüssigkeit durch das Zuleitungselement (104) zu steuern.

6. Vorrichtung nach einem der vorangehenden Ansprüche, wobei der Bürstenkopf (101) Borsten (103) unterschiedlicher Länge aufweist, um die Reinigung von Hinterschneidungen zu erleichtern, wobei die Borsten (103) bevorzugt bogenförmig in dem Borstenbereich (102) angeordnet sind.

7. Vorrichtung nach einem der vorangehenden Ansprüche, wobei das Zuleitungselement (104) eine Austrittsöffnung (108) aufweist, die innerhalb des Borstenbereichs (102) angeordnet ist.

8. Vorrichtung nach einem der vorangehenden Ansprüche, wobei das Ableitungselement (105) eine Ansaugöffnung (109) aufweist, die am proximalen Ende des Borstenbereichs (102) angeordnet ist.

9. Vorrichtung nach einem der vorangehenden Ansprüche, wobei die Vorrichtung eingerichtet ist, Reinigungsflüssigkeit aus dem Zuleitungselement (104) durch die Borsten (103) zu leiten und an der Spitze (110) der Borsten freizusetzen.

10. Vorrichtung nach einem der vorangehenden Ansprüche, wobei die Vorrichtung keine Pumpe aufweist.

11. Vorrichtung nach einem der vorangehenden Ansprüche, wobei das Zuleitungselement (104), und bevorzugt auch das Ableitungselement (105), durch das Verbindungselement (106) hindurchgeführt sind.

12. Vorrichtung nach einem der vorangehenden Ansprüche, weiterhin umfassend ein Griffelement (111), welches zwischen dem Verbindungselement (106) und dem Bürstenkopf (101) angeordnet ist, wobei das Griffelement (111) zur manuellen Führung der Vorrichtung durch einen Benutzer eingerichtet ist.

13. Vorrichtung nach Anspruch 12, weiterhin umfassend ein Stielelement (112), welches das Griffelement (111) mit dem Bürstenkopf (101) verbindet.

14. Vorrichtung nach Anspruch 13, wobei das Stielelement (112) eine Länge von 5 bis 10 cm aufweist und/oder einen Durchmesser von höchstens 10 mm aufweist.

15. Vorrichtung nach einem der vorangehenden Ansprüche, wobei das Zuleitungselement (104) einen flexiblen Schlauch umfasst und/oder eine Länge von mindestens 80 cm aufweist.
